# EUROPÄISCHE PATENTSCHRIFT

(11) **EP 2 044 172 B1**
(45) Veröffentlichungstag und Bekanntmachung des Hinweises auf die Patenterteilung: **22.06.2011**
(21) Anmeldenummer: 07765792.2
(22) Anmeldetag: 04.07.2007
(51) Int. Cl.: C09K 19/32, C09K 19/34

(54) **ANELLIERTE NAPHTHALINE**
FUSED NAPHTHALENES
NAPHTALINES ANNELÉES

(30) Priorität: 26.07.2006 DE 102006034430
(43) Veröffentlichungstag der Anmeldung: 08.04.2009
(73) Patentinhaber: Merck Patent GmbH, 64293 Darmstadt (DE)
(72) Erfinder: KRETZSCHMANN, Holger, 55452 Rümmelsheim (DE); EIDENSCHINK, Rudolf, 55129 Mainz (DE)
(86) Internationale Anmeldenummer: PCT/EP2007/056738
(87) Internationale Veröffentlichungsnummer: WO 2008/012180

(56) Entgegenhaltungen:
- WO-A-02/051963
- WO-A-2006/012965
- DE-A1- 10 313 700
- US-A- 5 648 021
- US-B1- 7 067 179

## Beschreibung

Gegenstand der vorliegenden Erfindung sind anellierte Naphthaline der allgemeinen Formel I 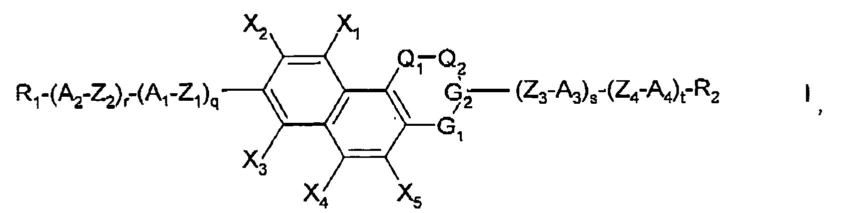 worin
- A₁,A₂,A₃,A₄: jeweils unabhängig voneinander einen unsubstituierten oder mit ein bis vier F-Atomen substituierten 1,4-Cyclohexylen-, 1,4-Cyclohexenylen oder 1,4-Cyclohexadienylenres, worin auch jeweils unabhängig voneinander eine oder zwei CH₂-Gruppen durch -O- oder -S- so ersetzt sein können, dass Heteroatome nicht direkt miteinander verknüpft sind, einen 1,4-Phenylenrest, der durch ein oder zwei Fluor- oder Chlor-Atome substituiert sein kann und in dem auch eine oder zwei CH-Gruppen durch N ersetzt sein können, einen 1,4-Bicyclo[2.2.2]octylenrest oder einen 2,6-Spiro[3.3]heptylenrest,
- Q₁-O₂: O-CH₂, CH₂-O, O-CF₂, CF₂-O, CH₂-CH₂, S-CF₂, CF₂-S, O-CO oder CO-O,
- G₁-G₂: CH₂-CH, CF₂-CH, CH=C, CF=C,
- R₁,R₂: jeweils unabhängig voneinander einen unsubstituierten oder mindestens einfach durch Halogen substituierten Alkylrest mit 1 bis 12 C-Atomen, worin auch eine oder mehrere nichtbenachbarte CH₂-Gruppen jeweils unabhängig voneinander durch -O-, -S-, -CO-, jeweils zwei benachbarte CH₂-Gruppen durch -CH=CH-, -CF=CF-, -COO-, -OOC-, -C≡C-, einen 1,2-Cyclopropanylenrest, der auch mit zwei F-Atomen substituiert sein kann, oder drei benachbarte CH₂-Gruppen durch einen 1,3-Cyclobutanyfenrest, der auch mit zwei F-Atomen substituiert sein kann, ersetzt sein können, F, Cl, -OCF₃, -OCHF₂, -CN, -NCS oder H, mit der Maßgabe, dass entweder nur R₁ oder nur R₂ H sein kann,
- X₁,X₂,: F,
- X₃, X_{4,} X₅: H
- Z_{1,}Z₂,Z₃,Z₄: jeweils unabhängig voneinander die Einfachbindung, -CH₂CH₂-, -CH₂O-, -OCH₂-, -CH₂CF₂-, -CF₂CH₂-,-CH₂CHF-, -CHFCH₂-, -CF₂O-, -OCF₂-, -COO-, -OOC-, -CH=CH-, -CF=CF-, -C≡C- und
- q,r,s,t: jeweils unabhängig voneinander 0 oder 1
bedeuten.

Die Erfindung betrifft weiterhin ein flüssigkristallines Medium, enthaltend mindestens eine Verbindung der allgemeinen Formel I, sowie die Verwendung eines solchen flüssigkristallinen Mediums in Anzeigeelementen, insbesondere elektrooptischen Anzeigeelementen.

Manche organischen Verbindungen gehen beim Erwärmen nicht direkt vom kristallinen in den flüssigen Zustand über, sondern durchlaufen innerhalb eindeutig begrenzter Temperaturbereiche eine oder mehrere zusätzliche Phasen. Diese Phasen haben richtungsabhängige physikalische Eigenschaften, sind aber wie Flüssigkeiten beweglich. Die physikalischen Merkmale solcher Phasen (im folgenden auch allgemein als flüssigkristalline Medien bezeichnet), wie der nematischen, cholesterischen, smektischen A oder der smektischen C Phase (vgl. P. G. deGennes and J. Prost, The Physics of Liquid Crystals, Clarendon Press, Oxford 1993) sind bekannt. Der molekulare Aufbau solcher für die Anwendung in der Elektrooptik typischen Verbindungen ist gekennzeichnet durch einen starren Grundkörper, enthaltend beispielsweise verknüpfte 1,4-Phenylen- oder 1,4-Cyclohexylengruppen oder auch kondensierte Ringsysteme, der an möglichst weit voneinander entfernten Stellen mit sog. mesogenen Resten , wie Alkyl-, Alkoxy- oder Cyangruppen substituiert ist.

Die flüssigkristallinen Medien, die in der Elektrooptik angewendet werden, bestehen aus Mischungen solcher Verbindungen, wobei auch solche Verbindungen Verwendung finden, die keinen enantiotropen Übergang von der kristallinen in eine flüsssigkristalline Phase haben. Im Falle der nematischen Phase hängen bekanntlich die optischen und dielektrischen Eigenschaften in erster Näherung linear vom Anteil der Mischungskomponenten ab. Allgemein sind die Verläufe der Werte für anisotrop-physikalische Größen für unterschiedliche Verbindungen oder Mischungen von Verbindungen ähnlich, wenn man sie über die sog. reduzierte Temperatur T_{R} (T_{R} = T_{M}/T_{K}, T_{M} ist die gemessene Temperatur, T_{K} Klärtemperatur, also die Temperatur des Übergangs von der nematischen in die isotrope Phase (alle in K)) aufträgt. Den Beitrag einer Komponente zu den anisotropen Eigenschaften kann man deshalb durch Vergleich bei der gleichen T_{R} ermitteln.

In den auf flüssigkristallinen Medien basierenden Anzeigeelementen (engl. Liquid Crystal Displays) wird bekanntlich die Veränderung eines optischen Eindrucks durch Anlegen eines elektrischen Feldes an Elektroden, zwischen denen sich ein flüssigkristallines Medium befindet, herbeigeführt. Besonders wichtig sind die wohlbekannten elektrooptischen Anzeigeelemente, die auf dem Prinzip der verdrillten nematischen Zelle (engl. Twisted Nematic Cell, TNC) beruhen. Durch Wechselwirkungen mit den Oberflächen der Elektroden werden die Moleküle einer nematischen Phase so orientiert, dass sie eine schraubenförmige Anordnung einnehmen. Hierdurch wird die Polarisationsebene durchtretenden Lichtes gedreht, so dass das Element zwischen zwei gekreuzten Polarisationsfolien transparent erscheint. Durch Anlegen einer Spannung an die Elektroden werden die Moleküle senkrecht orientiert. Voraussetzung hierfür ist eine positive dielektrische Anisotropie (Δε>0). Durch die bekannte Methode des sog. In Plane Switching (IPS), bei der die schraubenförmige Anordnung der Moleküle in eine parallele überführbar ist, sind besonders kontrastreiche Bilder erzeugbar. Besonders vorteilhaft erscheinen elektrooptische Anzeigeelemente, die ohne rückwärtige Beleuchtung und mit nur einer Polarisationsfolie auskommen. Solche reflektiven elektrooptischen Anzeigeelemente mit hoher Informationsdichte, niedriger Ansteuerspannung und einer geringen Abhängigkeit des Kontrastes vom Blickwinkel sind beschrieben in Y. Itoh et al., SID 98 Digest, 221. Bei diesen ist die Einhaltung eines optimalen Wertes für das Produkt aus der optischen Anisotropie Δn der nematischen Phase und seiner Schichtdicke d ausschlaggebend für eine geringe Abhängigkeit des Kontrastes vom Blickwinkel. Die Qualität der angezeigten Bilder oder alphanumerischen Angaben ist bekanntermaßen bei den genannten Anwendungen besser, je höher der spezifische elektrische Widerstand des flüssigkristallinen Mediums ist. Dies gilt insbesondere für den Betrieb von sog. Active Matrix Displays. Die Fähigkeit eines aus einem Dünnschichttransistor bestehenden Ansteuerelements, eine elektrische Spannung aufrechtzuerhalten, nachdem die Spannungsquelle abgeschaltet wurde, wird allgemein durch die sog. Voltage Holding Ratio (VHR) wiedergegeben. Für die immer wichtiger werdenden Anwendungen außerhalb geschlossener Räume werden flüssigkristalline Medien mit besonders hohen Klärpunkten benötigt. Die elektrische Ansteuerung der Pixel läßt sich bekanntlich vorteilhaft bei niedrigen Schwellenspannungen gestalten. Diese Schwellenspannungen werden nach bekannten Methoden bestimmt, wobei für nematlsche Phasen mit Δε>0 die sog. Frederiks-Schwelle (in Volt gemessen), für solche mit Δε<0 die sog. DAP-Schwelle (Deformation aufgerichteter Phasen) zu Grunde liegt. Die Zahl der möglichen Bildwechsel pro Zeiteinheit hängt von der Beweglichkeit der Moleküle in einem flüssigkristallinen Medium ab, die bei sinkender Temperatur rasch abnimmt. Für TNCs hängt die Schaltzeit direkt von der Rotationsviskosität des flüssigkristallinen Mediums ab, die wiederum mit den in üblichen Kapillar-Viskosimetern messbaren Werten korreliert. Bekannt sind auch elektrooptische Anzeigeelemente, die auf der Umorientierung ferroelektrischer oder antiferroetektnscher Schichten von smektischen C Phasen beruhen. Die bekannten Polymer Dispersed Liquid Crystals (PDLCs) bestehen aus in einem transparenten Polymer eingeschlossenen Tröpfchen aus flüssigkristallinen Medien, die das einfallende Licht abhängig von einer angelegten Spannung streuen. Mit flüssigkristallinen Medien, die eine negative dielektrische Anisotropie haben und dichroitische Farbstoffe enthalten, lassen sich elektrooptische Anzeigeelemente herstellen, die ebenfalls keine Polarisationsfolien benötigen. Besonders wichtig sind in neuerer Zeit elektrooptische Anzeigeelemente geworden, die auf der Modulation polarisierten Lichtes in nematisch flüssigkristallinen Medien mit negativer dielektrischer Anisotropie (Δε<0) nach der sog. VA-Technik (von engl, vertical alignment) beruhen.

Die in den oben genannten Anzeigeelementen verwendeten flüssigkristallinen Medien haben eine Reihe von Nachteilen, weil die als Mischungskomponenten verwendeten Verbindungen gegenüber Licht, Wärmeeinwirkung oder in elektrischen Feldern nicht ausreichend stabil sind. Auch lassen sich mit ihnen die für einen guten Kontrast nötigen optischen Anisotropien bei günstigen Betriebsspannungen und Schaltzeiten nur schwer erreichen.

In der Druckschrift US 7,067,179 B1 werden Tetrahydrophenanthrene mit Fluorsubstituenten als Flüssigkristallkomponente vorgeschlagen. Die Druckschrift WO 2006/012965 A1 offenbart 2,2-Difluorchmmanderivate als Flüssigkristallkomponente. DE 10313700 A1 offenbart fluorierte Oxaanthracene, US 5,648,021 offenbart fluorierte (9,10-Dihydro)-Phenanthrenderivate als Flüssigkristallmaterial. In der Druckschrift WO 02/051963 A1 werden flüssigkristalline Medien mit polyfluorierten Phenanthrenderivaten offenbart.

Die bekannten flüssigkristallinen Medien zeigen in elektrooptischen Anzeigeelementen noch verbesserungsbedürftige Eigenschaften. Insbesondere sind die erzielbaren Schwellenspannungen und die Schaltzeiten noch zu hoch.

Der Erfindung lag insbesondere die Aufgabe zu Grunde, eine Klasse stabiler Verbindungen bereitzustellen, welche die Herstellung elektrooptischer Anzeigeelemente mit niedrigen Schwellenspannungen und Schaltzeiten ermöglichen.

Es wurde überraschend gefunden, daß sich die anellierten Naphthaline der allgemeinen Formel I vorteilhaft als Komponenten flüssigkristalliner Medien eignen. Aus ihnen lassen sich stabile flüssigkristalline Medien mit besonders niedrigen Schwellenspannungen und niedrigen Schaltzeiten herstellen, die für die vorgenannten Anwendungen vorzüglich geeignet sind. Insbesondere eignen sich die erfindungsgemäßen anellierten Naphthaline auf Grund ihrer hohen VHR zur Herstellung von Active Matrix Displays. Sie haben hohe Klärtemperaturen und sind besonders stabil bei Einwirkung von Licht und bei Temperaturen oberhalb 120 °C. Die erfindungsmäßigen Verbindungen erweitern allgemein die Palette der flüssigkristallinen Substanzen zur Herstellung vorteilhafter flüssigkristalliner Medien für verschiedene, insbesondere für die vorstehend genannten Anwendungen.

Die erfindungsmäßigen anellierten Naphthaline der Formel I umfassen die 1,2,3,4-Tetrahydro-phenanthrene la 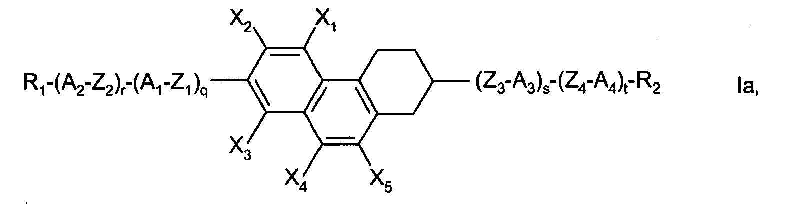 die 3,4-Dihydro-2H-benzo[h]chromene Ib 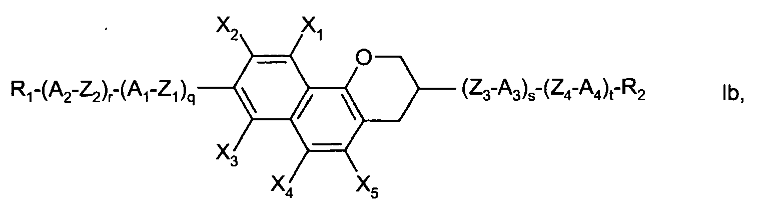 die 2,2-Difluor-3,4-dihydro-2H-benzo[h]chromene Ic 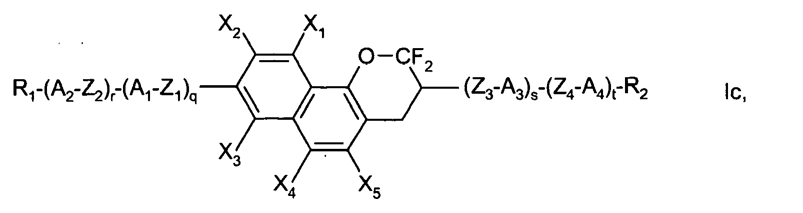 die 3,3,4,4-Tetrafluor-1,2,3,4-tetrahydro-phenanthrene Id 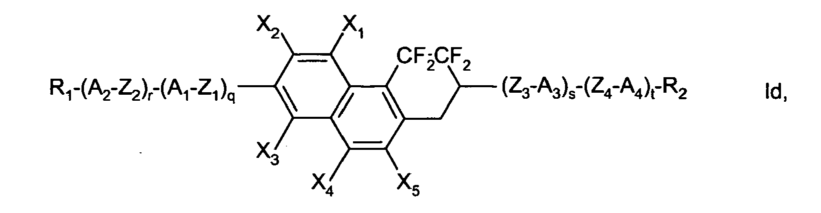 die 1-Fluor-3,4-dihydro-phenanthrene le 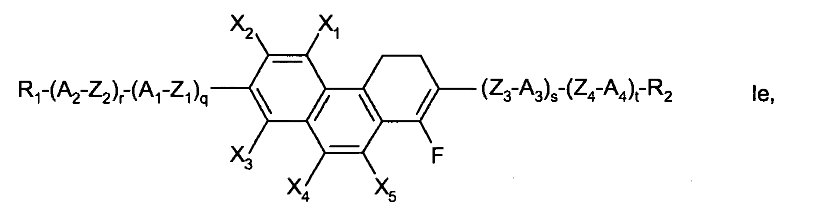 und die 2,2-Difluor-2H-benzo[h]chromene If 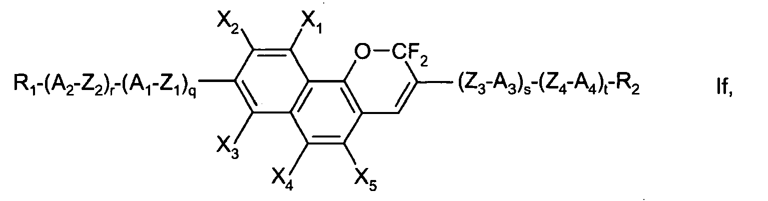 die allesamt bevorzugt sind. Besonders bevorzugt sind die Verbindungen der Formeln Ib und Ic

Vorzugsweise bedeuten in den Verbindungen der Formel I A₁, A₂, A₃, A₄ jeweils unabhängig voneinander unsubstituierte oder mit einem bis zwei F-Atomen substituierte 1,4-Phenylenreste oder 1,4-Cyclohexylenreste, in welchen auch eine CH₂-Gruppe jeweils unabhängig voneinander durch -O- ersetzt sein kann, wobei die 1,4-Cyclohexylen- und die 2,5-Tetrahydropyranylengruppen vorzugsweise trans-konfiguriert sind.

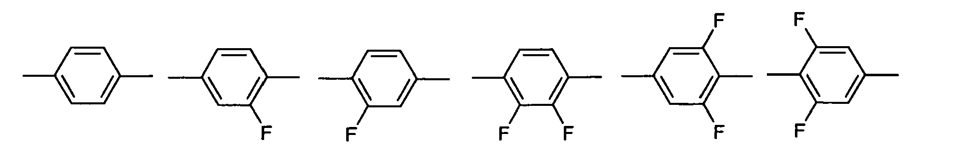

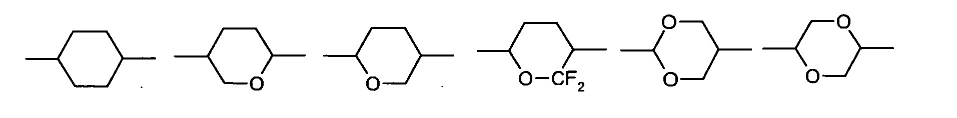

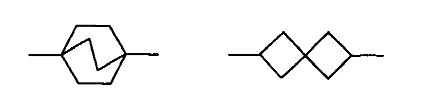

Die mit den Resten R₁ und R₂ der allgemeinen Formel I umfassten Alkylreste können geradkettig oder verzweigt sein. Bevorzugterweise sind sie geradkettig und bedeuten dann Methyl, Ethyl, Propyl, Butyl, Pentyl, Hexyl, Heptyl, Octyl, Nonyl, Decyl, Undecyl und Dodecyl. Sie können dann ausgedrückt werden durch -(CH₂)ᵤH. Wenn zwei CH₂-Gruppen hierin durch eine 1,2-Cyclopropylen-Gruppe oder drei CH₂-Gruppen durch eine 1,3-Cyclobutylen-Gruppe ersetzt ist, können diese Gruppen jeweils zwei F-Atome enthalten, z.B. also z.B

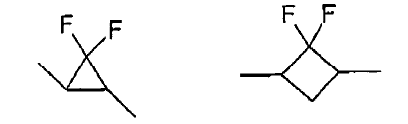

Wenn die Doppelbindung der ebenfalls umfaßten Alkenylreste nicht endständig sind, sind sie bevorzugterweise E(entgegen)-konfguriert. Die Bedeutungen von F und Cl für R₁ und R₂ sind ebenfalls bevorzugt, wenn diese Substituenten an einen Phenylrest gebunden sind.

Bevorzugt bedeutet Q₁-O₂ O-CH₂, O-CF₂ und CH₂CH₂, wobei O-CH₂ und O-CF₂ besonders bevorzugt sind.

G₁-G₂ bedeutet bevorzugt CH₂-CH.

Z₁, Z₂, Z_{3,} Z₄ in der allgemeinen Formel I bedeuten bevorzugterweise jeweils voneinander unabhängig die Einfachbindung, -CH₂CH₂-, -OCF₂- oder -CF₂O-, wovon die Einfachbindung besonders bevorzugt ist.

Die Indices q, r, s, t bedeuten jeweils unabhängig voneinander eins oder null, so dass Formel I null bis vier Reste der Bedeutung A₁, A₂, A₃, A₄ enthalten kann. Bevorzugt sind solche Verbindungen der Formel I, in denen die Summe aus q, r, s und t gleich 1 oder 2 ist, also neben dem Grundkörper des erfindungsgemäßen anellierten Naphthalins noch einen oder zwei ringförmige Reste der Bedeutung A₁, A₂, A₃, A₄ enthalten. Besonders bevorzugt sind solche Verbindungen, für welche die Summe aus q und r 1 oder 2 ist und gleichzeitig die Summe aus s und t null oder 1 ist.

Die Formel I umfasst auch optisch aktive Verbindungen und deren Racemate, wenn in ihr G₁-G₂ keine Doppelbindung enthält. Erstere können durch gezielte asymmetrische Synthesen oder aus den optisch nicht aktiven erfindungsmäßigen Verbindungen durch säulenchromatographische Trennung an chiralem Trägermaterial, etwa Cyclodextrinen, gewonnen werden. Die optisch aktiven Verbindungen der Formel I eignen sich insbesondere für Phasen mit ferroelektrischen und antiferroelektrischen Eigenschaften sowie zur Herstellung cholesterischer Phasen.

Auch sind solche Verbindungen der Formel I umfaßt, in denen die gebundenen Elemente eine von der natürlichen abweichende Verteilung ihrer Isotopen haben.

Im Folgenden sind einige Verbindungen der Formel aufgeführt, wobei R₁ und R₂ die angegebenen Bedeutungen haben.

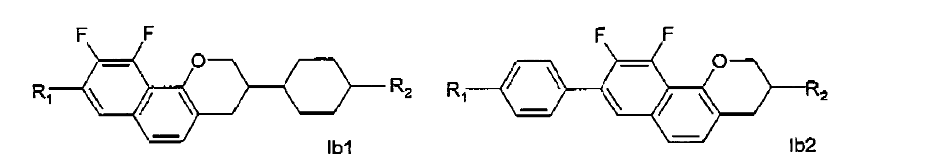

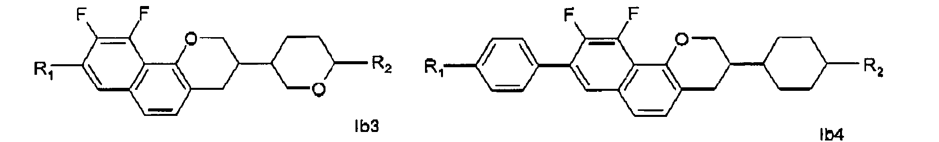

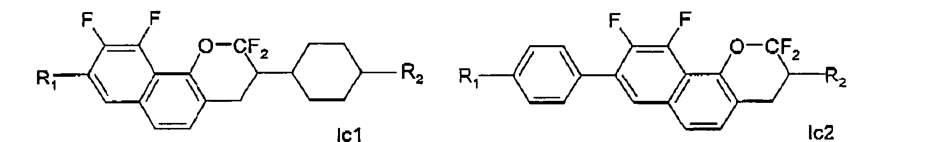

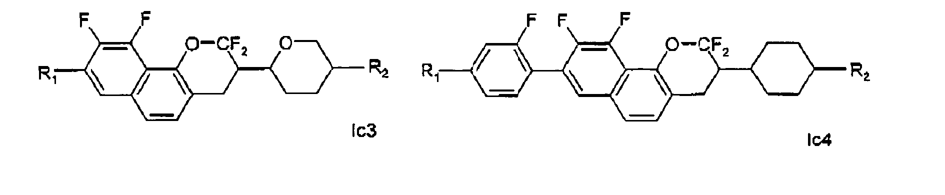

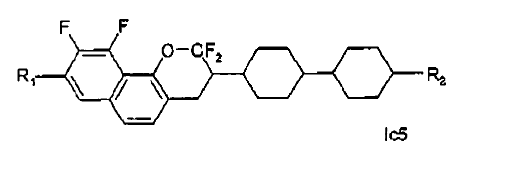

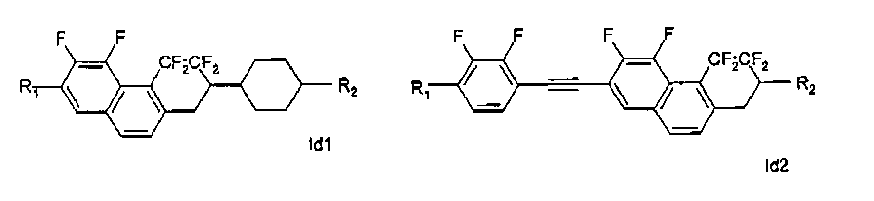

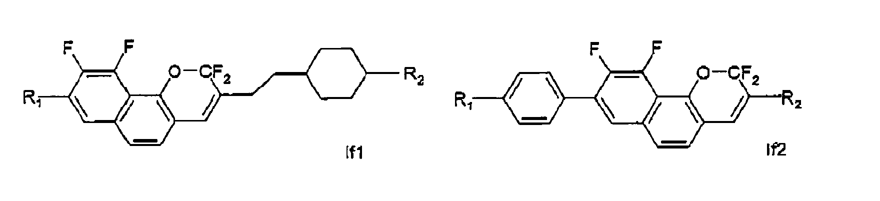

Die Verbindungen der allgemeinen Formel I werden nach allgemein bekannten Methoden hergestellt. Diese sind beispielsweise dem Sammelwerk Houben-Weyl, Methoden der organischen Chemie, Georg Thieme Verlag, Stuttgart und sonstigen, dem Fachmann zugänglichen Veröffentlichungen zu entnehmen Experimentelle Details zu den im Folgenden aufgeführten Suzuki- und Heck-Kupplungen sind in der Monographie J. Tsuji, Palladium Reagents and Catalysts, John Wiley & Sons, Chichester, 2004 und der dort aufgeführten Literatur zu entnehmen.

Günstig ist die Verknüpfung eines mit Iod, Brom oder Chlor (hier mit Y gekennzeichnet) substituierten anellierten Naphthalins der Form 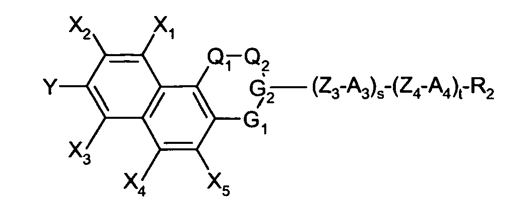 abgekürzt als Y-Ar, durch eine allgemein bekannte Suzuki-Kupplung mit einer Boronsäure.

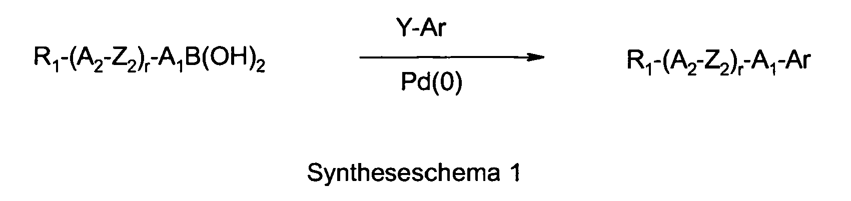

Auch kann der B(OH)₂-Rest am Naphthalin-Ring fixiert werden (vgl. Syntheseschema 3). Neben Suzuki-Kupplungen mit aromatischen Halogenverbindungen zu Verbindungen der allgemeinen Formel I oder ihren Vorstufen ist auch durch Kochen mit H₂O₂-Lösung die Überführung in ein Phenol und die anschließende Umsetzung mit einem Alkylbromid in Anwesenheit einer Base in eine erfindungsgemäße Alkoxyverbindung möglich.

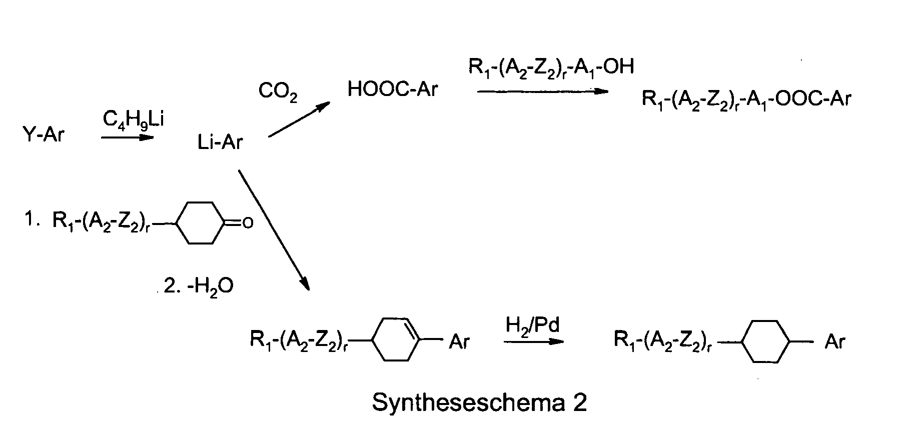

Ein Halogen-Metall-Austausch (z.B. Brom-Lithium-Austausch) mit anschließender Addition an eine C=O Bindung führt nach wohlbekannten Folgereaktionen ebenfalls zu Verbindungen der allgemeinen Formel I (Syntheseschema 2).

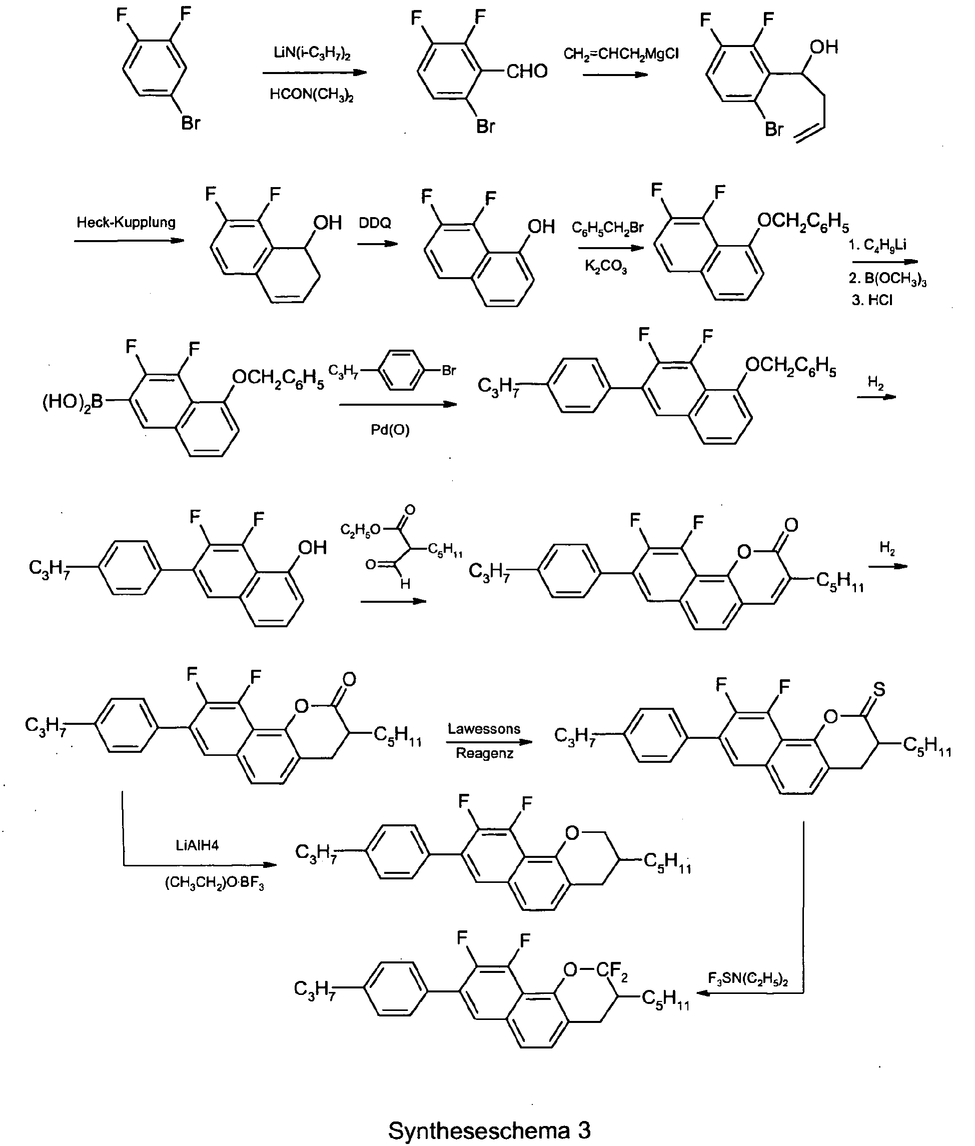

In Syntheseschema 3 ist beispielhaft eine Folge von allgemein bekannten Syntheseschritten zu Verbindungen der Teilformeln Ib und Ic aufgeführt. Der aus käuflichem 3,4-Difluor-brombenzol durch Metallierung mit Lithiumdiisopropylamid (LDA) in Tetrahydrofuran (THF) und anschließender Umsetzung mit Dimethylformamid erhältliche substituierte Benzaldehyd wird mit Allylmagnesiumchlorid in einen Allylbenzylalkohol überführt, der nach Zusatz von [P(o-Tolyl)₃]PdCl₂ eine Heck-Kupplung zu einem Hydroxy-dihydronaphthalin eingeht, das anschließend durch ein Oxidationsmittel aromatisiert wird. Benzylierung mit Benzylbromid in Aceton bei Anwesenheit von Kaliumcarbonat führt zu einem Benzylether, der sich in THF lithiieren lässt. Aus der Lithium-Verbindung entsteht durch Reaktion mit Borsäuretrimethylether und anschließendem Ansäuern in allgemein bekannter Weise eine Boronsäure, die durch Umsetzung mit Arylhalogeniden der Form R₁(A₂-Z₂)ᵣ-A₁-Hal, wobei R₁, A₁, A₂ und Z₁ die innerhalb der vorliegenden Erfindung geltenden Bedeutungen haben, und Hal Cl, Br oder I bedeuten, mit bekannten geeigneten Katalysatoren zu Aryl-naphthalinen umgesetzt werden. Durch katalytische Hydrogenolyse wird ein 1-Naphthol erzeugt, das mit einem substituierten Formylessigsäuremethylester (vgl. K. Chebaane et al., Bull. Soc. Chim. France 1975, 2516) zu dem von der Erfindung umfassten Benzocumarin umgesetzt wird, das durch katalytische Hydrierung in das ebenfalls von der Formel I umfasste Dihydro-Derivat umgewandelt werden kann. Letztere Verbindung kann in ebenfalls allgemein bekannter Weise durch Reduktion mit LiAlH₄ oder Nah4 und BF₃-Etherat in eine Verbindung der Formel Ib oder durch Umsetzung mit Lawessons-Reagenz und sich anschließender Umwandlung der CS- in eine CF₂-Gruppe mit Diethylaminoschwefeltrifluorid (DAST) (vgl. S. Scheibye et al., Tetrahedron 35, 1339 (1979)) in eine Verbindung der allgemeinen Formel Ic überführt werden.

Von dem Dihydrocumarin aus dem Schema 3 führt ein Weg zu Verbindungen der Formel If (Syntheschema 4).

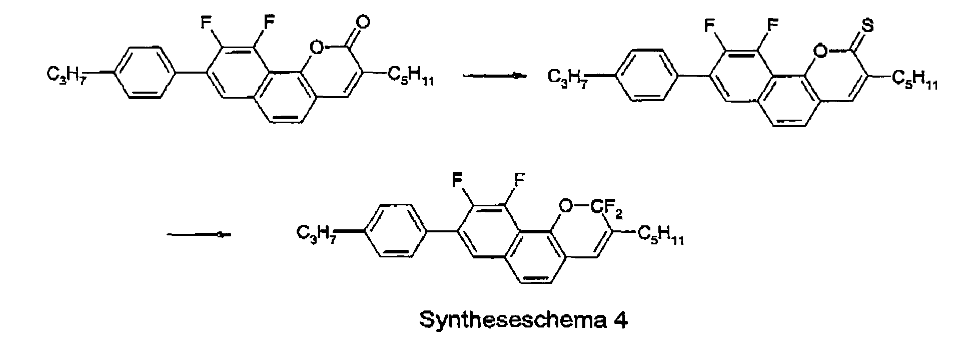

Verbindungen der Formel Ia sind nach Syntheseschema 5 aus substituierten Naphthalinen durch Ringschluss mittels einer Friedel-Crafts-Acylierung (vgl. B. Hulin, M. Koreeda, J. Org. Chem. 49, 207 (1984)) zugänglich (zum Vergleich; Fluorsubstituenten hier nicht gemäß Ansprüchen).

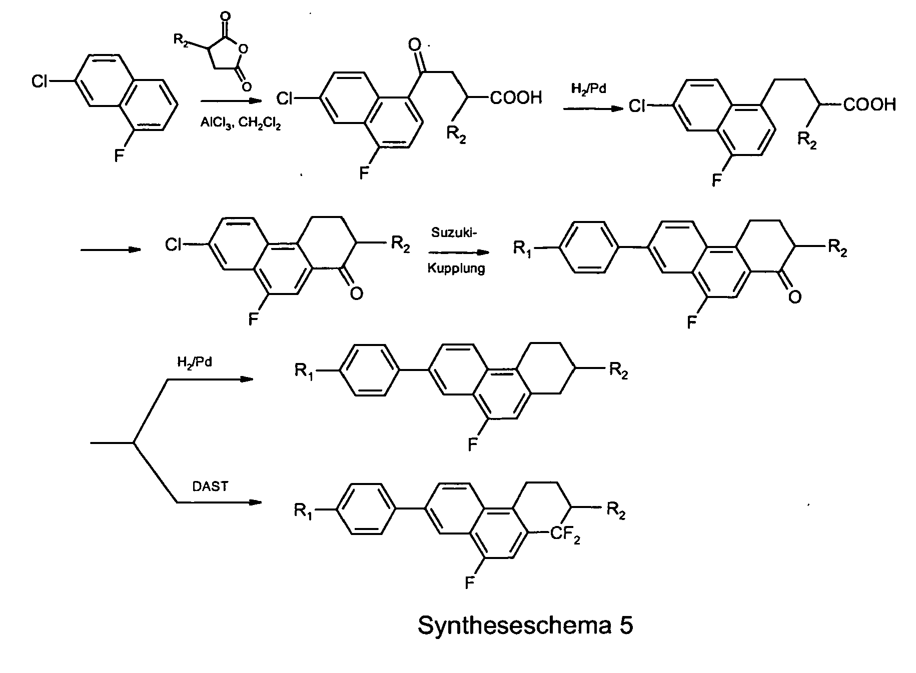

Das ebenfalls von der Erfindung umfaßte flüssigkristalline Medium besteht aus mindestens zwei flüssigkristallinen Komponenten und enthält als Komponente mindestens eine Verbindung der allgemeinen Formel I. Typischerweise enthält es eine bis fünf, bevorzugterweise zwei bis vier Verbindungen der Formel I. Als weitere Mischungskomponenten des erfindungsmäßigen flüssigkristallinen Mediums können die allgemein bekannten mesogenen, d.h. in reiner Form oder in Mischung mit anderen Komponenten zur Bildung flüssigkristalliner Phasen befähigten, Verbindungen dienen. Einige solcher Verbindungen sind z.B. in DE 19804894 und WO 2005/037957 aufgeführt. Die wichtigsten sind nach der allgemeinen Formel II

U₁-(B₁-Y₁)ₘ-(B₂-Y₂)ₙ-B₃-U₂ II

aufgebaut. Hierin bedeuten
- B₁ bis B₃: jeweils unabhängig voneinander unsubstituierte oder durch Halogen oder -CN substituierte 1,4-Cyclohexylen-, in denen eine oder zwei CH₂-Gruppen durch -O- ersetzt sein können, 4-Bicyclo[2.2.2]octylen-, 1,4-Phenylen- und 2,5-Pyrimidinylenreste sowie 2,6-Naphthalinylen-, 1,2,3,4- Tetrahydronaphthalin-2,4-ylen- oder Indan-2,5-ylenreste
- U₁, U₂: unabhängig voneinander unsubstituierte oder mit mindestens einem Halogenatom substituierte Alkyl- oder Alkenylreste mit 1 bis 12 C-Atomen, in denen eine oder mehrere nichtbenachbarte CH₂-Gruppen durch -O- oder -S- ersetzt sein können, -CN, OCHF₂, -OCF₃, -SF₅, -F, -Cl, -OCH=CF₂, -N=C=S
- Y₁, Y₂: unabhängig voneinander -CH₂CH₂-, -COO-, -CH=CH-, -OCF₂-, die Einfachbindung und
- m, n: 0, 1 oder 2.

Beispielhaft als Mischungskomponenten seien aufgeführt, wobei die aufgeführten Alkylgruppen stellvertretend für Alkylgruppen mit 1 bis 12 C-Atomen stehen, in denen 1 oder 2 CH₂-Gruppen durch -O- oder -CH=CH- ersetzt sind:

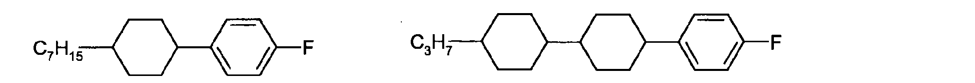

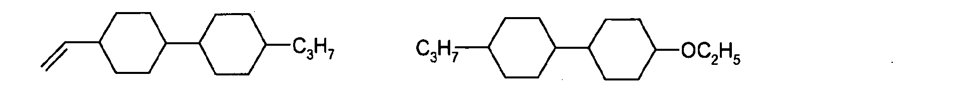

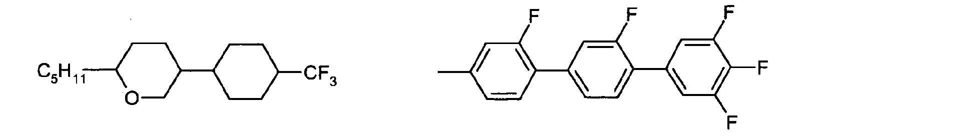

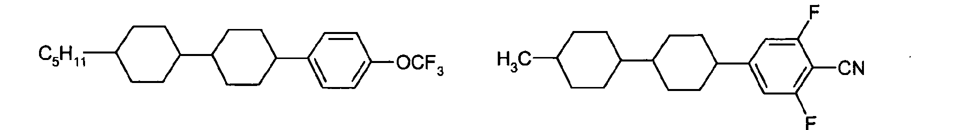

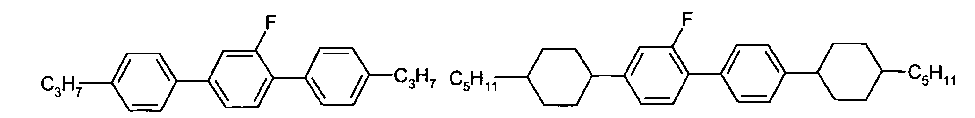

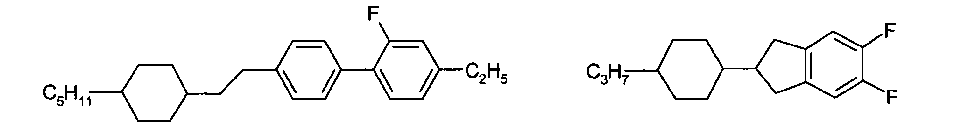

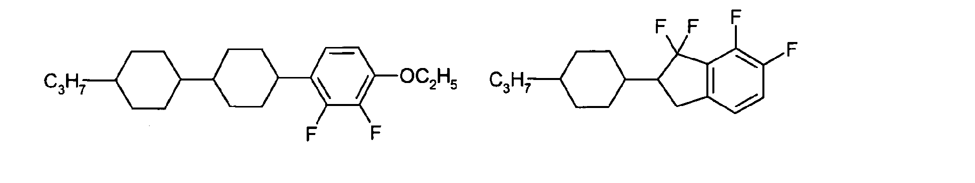

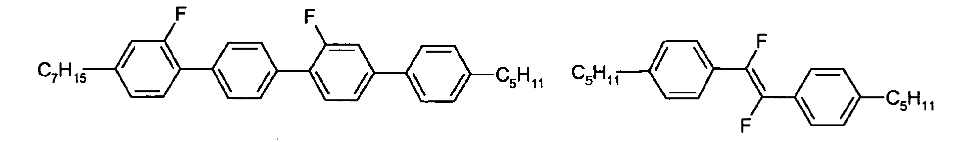

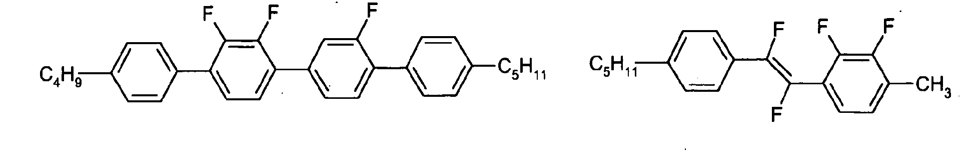

Es können aber auch weitere Mischungskomponenten, wie dichroitische Farbstoffe, nicht von der Erfindung umfasste chirale Verbindungen, Leitsalze sowie gelbildende Polymere oder amorphe Festkörper, wie pyrogene Kieselsäuren, zugesetzt werden. Auch der Zusatz von Oxidationsinhibitoren ist möglich.

Die erfindungsmäßigen flüssigkristallinen Medien werden hergestellt, indem man die Komponenten zusammen in einem geeigneten Glasgefäß auf 80°C erwärmt, durch Rühren vermischt und auf Raumtemperatur abkühlen läßt. Der Anteil der anellierten Naphthaline der allgemeinen Formel I im erfindungsmäßigen flüssigkristallinen Medium kann zwischen 1 und 99 Masseprozent liegen. Bevorzugterweise liegt er zwischen 10 und 90 Masseprozent und in besonders bevorzugter Weise zwischen 15 und 50 Masseprozent.

Die folgenden Beispiele sollen die Erfindung erläutern, ohne sie zu begrenzen. Alle Prozentangaben bedeuten Masseprozent. Die Temperaturen sind nachstehend in Grad Celsius angegeben. Fp. bedeutet Schmelztemperatur, h Stunde, min Minute. Kr bedeutet kristalline, Sm smektische, Ne nematische und Is isotrope Phase. Die zwischen diesen Bezeichnungen stehenden Zahlen geben die Temperatur an, bei der ein Übergang zwischen den bezeichneten Phasen erfolgt. IR steht für Infrarotspektrum, KBr bedeutet, dass das Spektrum mit einem üblichen Kaliumbromid-Pressling aufgenommen worden ist. Wenn nichts anderes vermerkt ist, sind die Alkylreste n-Alkylreste.

### Beispiele 1 bis 4

Gemäß Syntheseschema 3 wird kommerziell erhältliches 3,4-Difluor-brombenzol in THF bei -60 °C unter Stickstoff mit der 1,1-fachen molaren Menge an festem LDA versetzt.

Zu der entstandenen Lösung der Aryl-Lithium-Verbindung wird bei -60 °C die zur Bromverbindung 1,15-fach molare Menge an N,N-Dimethyl-formamid (DMF)gefügt. Nach 3 h wird auf Raumtemperatur erwärmt, mit Wasser versetzt und der 2-Brom-5,6-difluor- benzaldehyd durch Ausschütteln der wässrigen Phase mit Diethylether, Trocknung der organischen Phase mit Natriumsulfat und Abdestillieren des Lösungsmittels (im Folgenden als übliche Aufarbeitung bezeichnet) als öliger Rückstand isoliert. Seine Reaktion mit einer äquimoleren Menge an Allylmagnesiumchlorid in THF bei Raumtemperatur liefert nach Versetzen mit gesättigter wässriger Ammoniumchlorid-Lösung und üblicher Aufarbeitung rohes 4-(2-Brom-5,6-difluorphenyl)-4-hydroxy-buten-1. Die säulenchromatographische Reinigung (Kieselgel 60 (Merck KGaA), Fließmittel Toluol mit 10 Voumenprozent Isopropanol) liefert nach Eindampfen der Hauptfraktion reines Produkt. Die intramolekulare Heck-Kupplung erfolgt bei 140°C in DMF nach Zusatz von Natriumacetat, Benzyl-triethylammoniumchlorid und [P(o-Tolyl)₃]PdCl₂ als Katalysator. Nach dem Abkühlen auf Raumtemperatur und Verdünnen mit Wasser wird wie üblich aufgearbeitet. Das Rohmaterial wird durch Kochen mit 2,3-Dichlor-5,6-dicyano-benzochinon in Dichlormethan in allgemein bekannter Weise zum 1-Naphthol aromatisiert, das dann in einer Suspension eines Überschusses von Kaliumcarbonat in Aceton mit Benzylbromid in den Benzylether überführt wird. Dieser wird in THF gelöst, bei -60 °C mit Butyl-lithium in allgemein bekannter Weise lithiiert und die gelöste metallorganische Verbindung anschließend mit Borsäuretrimethylester versetzt. Die nach dem Ansäuern mit verdünnter Salzsäure ausfallende Boronsäure wird aus n-Heptan umkristallisiert. Sie wird anschließend in THF gelöst und nach Zusatz von 4-Propyl-brombenzol und einer katalytischen Menge von Pd[P(C₆H₅)₄] nach Suzuki gekuppelt. Die allgemein bekannte Hydrogenolyse mit Wasserstoff in Anwesenheit von Palladium (5 % Pd auf Kohle) liefert das entsprechende 1-Naphthol. Dieses wird zusammen mit der 1,1 fach molaren Menge von Pentyl-formylessigsäureethylester bei etwa -10°C in einem ausreichenden Volumen Ethanols gelöst und die Lösung mit gasförmigem Chlorwasserstoff gesättigt. Es wird auf Raumtemperatur erwärmt und ca. 20 h stehen gelassen. Danach wird auf Eis gegossen und das sich abscheidende 3-Pentyl-8-(4-propyl-phenyl)-benzo[h]chromen-2-on (Vergleichsbeispiel 1) aus Ethanol umkristallisiert. Die Hydrierung unter Pd-Katalyse in THF führt zu 3-Pentyl-8-(4-propyl-phenyl)-3,4-dihydro-benzo[h]chromen-2-on (Vergleichsbeispiel 2) das nach Abfiltrieren des Katalysators und Abdestillieren des Lösungsmittels durch Umkristallisation aus Ethanol gereinigt wird. Hieraus wird in allgemein bekannter Weise durch Umsetzung mit LiAlH₄ und Bortrifluorid-Etherat (vgl. G. R. Pettit et al., J. Org. Chem. 26, 4773 (1961)) das 3-Pentyl-8-(4-propyl-phenyl)-3,4-dihydro-benzo[h]chromen (Vergleichsbeispiel 3) erzeugt. Die Verbindung von Beispiel 2 wird durch Umsetzung mit 2,4-Bis(4-methoxyphenyl)-1,3-dithia-2,4-diphosphetane-2,4-disulfid (Lawessons-Reagenz) in das entsprechende Thion überführt (vgl. P. S. Pederson et al., Bull. Soc. Chim. Belg. 87, 293 (1978)), woraus in allgemein bekannter Weise durch Umsetzung mit DAST in Dichlormethan das 2,2-Difluor-3-pentyl-8-(4-propyl-phenyl)-3,4-dihydro-benzo[h]chromen (Vergleichsbeispiel 4) entsteht. Die Verbindungen der Beispiele 3 und 4 werden jeweils nach üblicher Aufarbeitung zur Reinigung aus Ethanol umkristallisiert.

### Vergleichsbeispiel 5

Eine weitere beispielhafte Verbindung kann in wenigen Synthesestufen nach Umsetzung von Ethyl-2-(4-ethoxyphenyl)-3-oxopropanoat mit 1,6-Dihydroxynaphthalin nach folgendem Syntheseschema (vgl. D. F. Taber et al., J. Org. Chem. 54, 3831 (1989)) hergestellt werden:

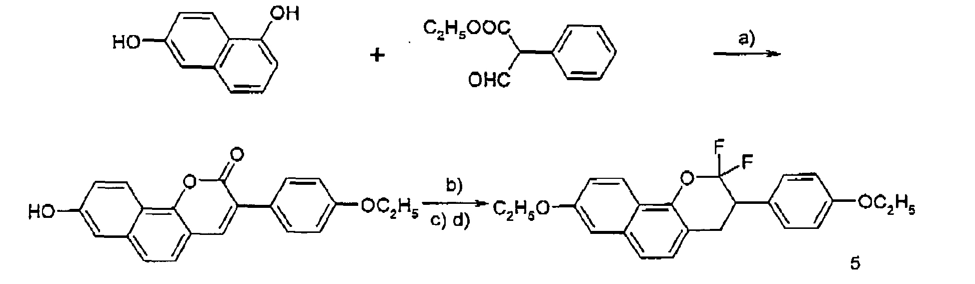

### Beispiele 1 bis 4

Nach allgemein bekannten Methoden werden ebenfalls synthetisiert:

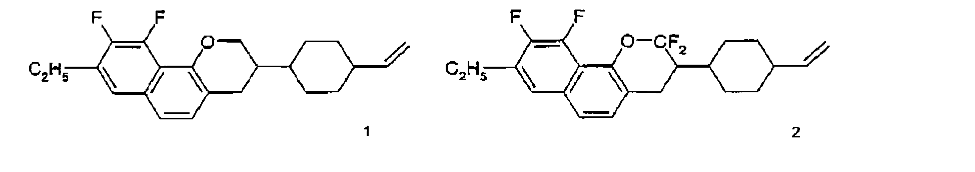

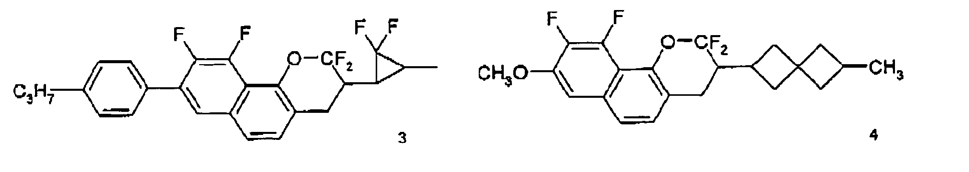

### Beispiel 5

Eine Mischung bestehend aus

| | |
|---|---|
| 15 % | 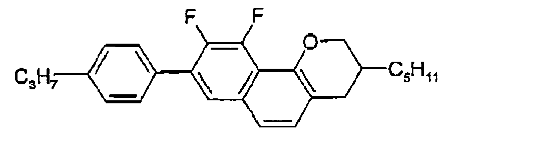 |
| 15% | 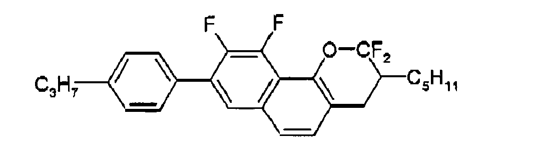 |
| 30% | 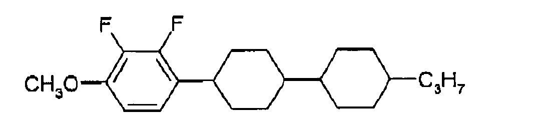 |
| 30% | 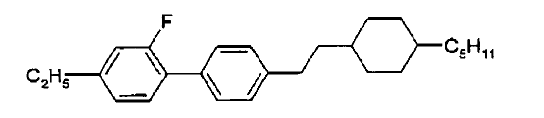 |
| 10% | 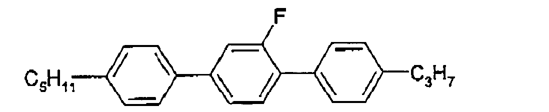 |

ist bei Raumtemperatur nematisch und hat eine DAP-Schwelle von <5 V Sie ist für elektrooptische Anzeigeelemente der VA-Technik geeignet.

## Patentansprüche

1. Anellierte Naphthaline der allgemeinen Formel I 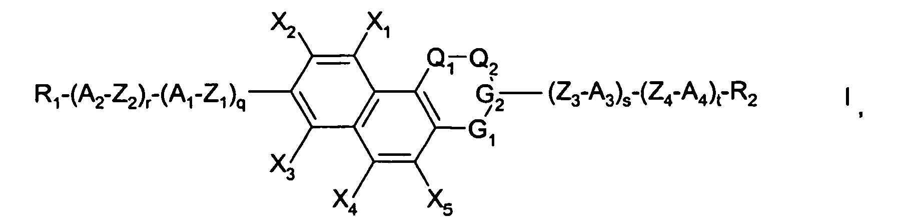 worin
A₁, A₂, A₃, A₄ jeweils unabhängig voneinander einen unsubstituierten oder mit ein bis vier F-Atomen substituierten 1,4-Cyclohexylen-, 1,4-Cyclohexenylen oder 1,4-Cyclohexadienylenrest, worin auch jeweils unabhängig voneinander eine oder zwei CH₂-Gruppen durch -O- oder -S- so ersetzt sein können, dass Heteroatome nicht direkt miteinander verknüpft sind, einen 1,4-Phenylenrest, der durch ein oder zwei Fluor- oder Chlor-Atome substituiert sein kann und in dem auch eine oder zwei CH-Gruppen durch N ersetzt sein können, einen 1,4-Bicyclo[2.2.2]octylenrest oder einen 2,6-Spiro[3.3]heptylenrest,
Q₁-Q₂ O-CH₂, CH₂-O, O-CF₂, CF₂-O, CH₂-CH₂, S-CF₂, CF₂-S, O-CO oder CO-O,
G₁-G₂ CH₂-CH, CF₂-CH, CH=C, CF=C,
R₁, R₂ jeweils unabhängig voneinander einen unsubstituierten oder mindestens einfach durch Halogen substituierten Alkylrest mit 1 bis 12 C-Atomen, worin auch eine oder mehrere nichtbenachbarte CH₂-Gruppen jeweils unabhängig voneinander durch -O-, -S-, -CO-, jeweils zwei benachbarte CH₂-Gruppen durch -CH=CH-, -CF=CF-, -COO-, -OOC-, -C≡C-, einen 1,2-Cyclopropanylenrest, der auch mit zwei F-Atomen substituiert sein kann, oder drei benachbarte CH-Gruppen durch einen 1,3-Cyclobutanylenrest, der auch mit zwei F-Atomen substituiert sein kann, ersetzt sein können, F, Cl, -OCF₃, -OCHF₂, -CN, -NCS oder H, mit der Maßgabe, dass entweder nur R₁ oder nur R₂ H sein kann,
X₁, X₂ F,
X₃, X₄, X₅ H,
Z₁,Z₂,Z₃,Z₄ jeweils unabhängig voneinander die Einfachbindung, -CH₂CH₂-, -CH₂O-, -OCH₂-, -CH₂CF₂-, -CF₂CH₂-,-CH₂CHF-, -CHFCH₂-, -CF₂O-, -OCF₂-, -COO-, -OOC-, -CH=CH-, -CF=CF-, -C=C- und
q,r,s,t jeweils unabhängig voneinander 0 oder 1
bedeuten.

2. Anellierte Naphthaline nach Anspruch 1, **dadurch gekennzeichnet, dass** Q1-Q2 die Bedeutung O-CH₂ hat.

3. Anellierte Naphthaline nach Anspruch 1, **dadurch gekennzeichnet, dass** Q₁-Q₂ die Bedeutung O-CF₂ hat.

4. Anellierte Naphthaline nach Ansprüchen 1 und 2 der allgemeinen Formel 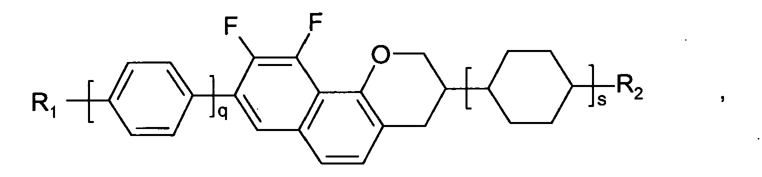 worin R₁. R₂, q und s die angegebene Bedeutung haben.

5. Anellierte Naphthaline nach Ansprüchen 1 und 3 der allgemeinen Formel 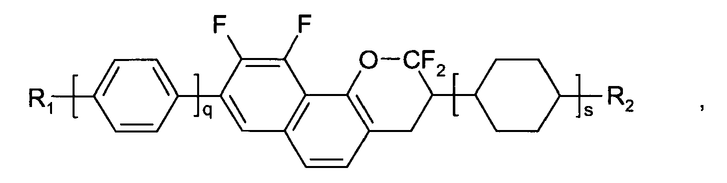 worin R₁, R₂, q, und s die angegebene Bedeutung haben.

6. Verwendung von Verbindungen der Formel I nach einem der vorangehenden Ansprüche als Komponenten flüssigkristalliner Medien.

7. Flüssigkristallines Medium mit mindestens zwei flüssigkristallinen Komponenten, **dadurch gekennzeichnet, dass** es mindestens eine Verbindung nach einem der Ansprüche 1 bis 5 enthält.

8. Elektrooptisches Anzeigeelement, **dadurch gekennzeichnet, dass** es ein flüssigkristallines Medium nach Anspruch 7 enthält.

## Claims

1. Fused naphthalenes of the general formula I 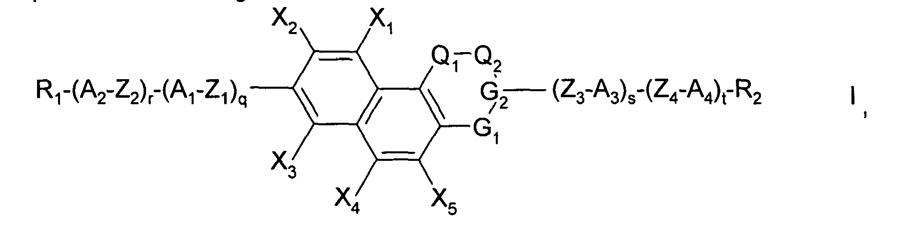 in which
A₁, A₂, A₃, A₄ each, independently of one another, denote a 1,4-cyclohexylene, 1,4- cyclohexenylene or 1,4-cyclohexadienylene radical which is unsubstituted or substituted by one to four F atoms and in which one or two CH₂ groups may each be replaced, independently of one another, by -O- or -S- in such a way that heteroatoms are not linked directly to one another, a 1,4-phenylene radical, which may be substituted by one or two fluorine or chlorine atoms and in which, in addition, one or two CH groups may be replaced by N, or denote a 1,4-bicyclo[2.2.2]octylene radical or a 2,6-spiro[3.3]heptylene radi- cal,
Q₁-Q₂ denotes O-CH₂, CH₂-O, O-CF₂, CF₂-O, CH₂-CH₂, S-CF₂, CF₂-S, O-CO or CO-O,
G₁-G₂ denotes CH₂-CH, CF₂-CH, CH=C, CF=C,
R₁, R₂ each, independently of one another, denote an alkyl radical having 1 to 12 C atoms which is unsubstituted or at least monosubstituted by halogen and in which one or more non-adjacent CH₂ groups may each, independently of one another, be replaced by -O-, -S-, -CO-, two adjacent CH₂ groups may each be replaced by -CH=CH-, -CF=CF-, -COO-, -OOC-, -C≡C-, a 1,2- cyclopropanylene radical, which may also be substituted by two F atoms, or three adjacent CH₂ groups may be replaced by a 1,3-cyclobutanylene radi- cal, which may also be substituted by two F atoms, or denote F, Cl, -OCF₃, -OCHF₂, -CN, -NCS or H, with the proviso that either only R₁ or only R₂ can be H,
X₁, X₂ denote F,
X₃, X₄, X₅ denote H,
Z₁, Z₂, Z₃, Z₄ each, independently of one another, denote a single bond, -CH₂CH₂-, -CH₂O-, -OCH₂-, -CH₂CF₂-, -CF₂CH₂-, -CH₂CHF-, -CHFCH₂-, -CF₂O-, -OCF₂-, -COO-, -OOC-, -CH=CH-, -CF=CF-, -C≡C-, and
q, r, s, t each, independently of one another, denote 0 or 1.

2. Fused naphthalenes according to Claim 1, **characterised in that** Q₁-Q₂ has the meaning O-CH₂.

3. Fused naphthalenes according to Claim 1, **characterised in that** Q₁-Q₂ has the meaning O-CF₂.

4. Fused naphthalenes according to Claims 1 and 2 of the general formula 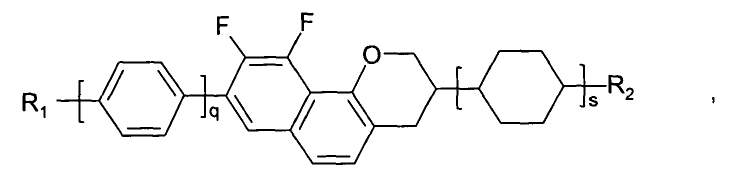 in which R₁, R₂, q and s have the meaning indicated.

5. Fused naphthalenes according to Claims 1 and 3 of the general formula 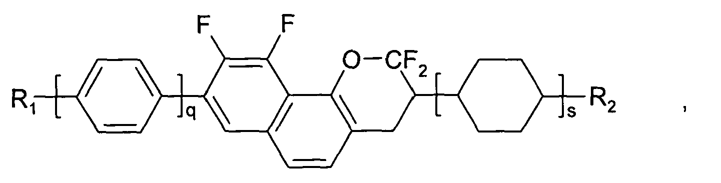 in which R₁, R₂, q and s have the meaning indicated.

6. Use of compounds of the formula I according to one of the preceding claims as components of liquid-crystalline media.

7. Liquid-crystalline medium having at least two liquid-crystalline components, **characterised in that** it comprises at least one compound according to one of Claims 1 to 5.

8. Electro-optical display element, **characterised in that** it contains a liquid-crystalline medium according to Claim 7.

## Revendications

1. Naphtalènes fusionnés de la formule générale I 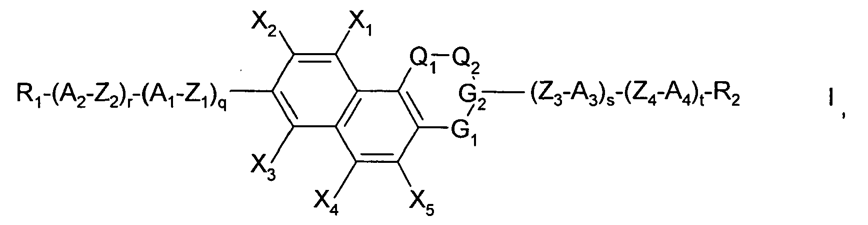 dans laquelle
A₁, A₂, A₃, A₄ représentent, chacun indépendamment des autres, un radical 1,4-cyclo- hexylène, 1,4-cyclohexénylène ou 1,4-cyclohexadiénylène, lequel est non substitué ou substitué par un à quatre atomes de F et où un ou deux groupes CH₂ peuvent chacun être remplacés, indépendamment l'un de l'autre, par -O- ou -S- de telle sorte que des hétéroatomes ne soient pas liés directe- ment les uns aux autres, un radical 1,4-phénylène, lequel peut être substitué par un ou deux atomes de fluor ou de chlore et où, en addition, un ou deux groupes CH peuvent être remplacés par N, ou représentent un radical 1,4- bicyclo[2.2.2]octylène ou un radical 2,6-spiro[3.3]heptylène,
Q₁-Q₂ représente O-CH₂, CH₂-O, O-CF₂, CF₂-O, CH₂-CH₂, S-CF₂, CF₂-S, O-CO ou CO-O,
G₁-G₂ représente CH₂-CH, CF₂-CH, CH=C, CF=C,
R₁, R₂ représentent, chacun indépendamment des autres, un radical alkyle compor- tant de 1 à 12 atomes de C, lequel est non substitué ou au moins monosubs- titué par halogène et où un ou plusieurs groupes CH₂ non adjacents peuvent, chacun indépendamment les uns des autres, être remplacés par -O-, -S-, -CO-, deux groupes CH₂ adjacents peuvent chacun être remplacés par -CH=CH-, -CF=CF-, -COO-, -OOC-, -C≡C-, un radical 1,2-cyclopropan- ylène, lequel peut également être substitué par deux atomes de F, ou trois groupes CH₂ adjacents peuvent être remplacés par un radical 1,3-cyclo- butanylène, lequel peut également être substitué par deux atomes de F, ou représentent F, Cl, -OCF₃, -OCHF₂, -CN, -NCS ou H, sous réserve que soit seulement R₁, soit seulement R₂ peut être H,
X₁, X₂ représentent F,
X₃, X₄, X₅ représentent H,
Z₁, Z₂, Z₃, Z₄ représentent, chacun indépendamment des autres, une liaison simple, -CH₂CH₂-, -CH₂O-, -OCH₂-, -CH₂CF₂-, -CF₂CH₂-, -CH₂CHF-, -CHFCH₂, -CF₂O-, -OCF₂-, -COO-, -OOC-, -CH=CH-, -CF=CF-, -C≡C-, et
q, r, s, t représentent, chacun indépendamment des autres, 0 ou 1.

2. Naphtalènes fusionnés selon la revendication 1, **caractérisés en ce que** Q₁-Q₂ présente la signification O-CH₂.

3. Naphtalènes fusionnés selon la revendication 1, **caractérisés en ce que** Q₁-Q₂ présente la signification O-CF₂.

4. Naphtalènes fusionnés selon les revendications 1 et 2 de la formule générale 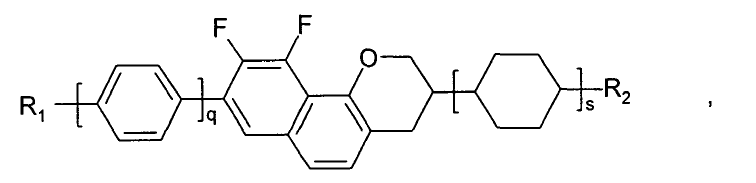 dans laquelle R₁, R₂, q et s présentent la signification indiquée.

5. Naphtalènes fusionnés selon les revendications 1 et 3 de la formule générale 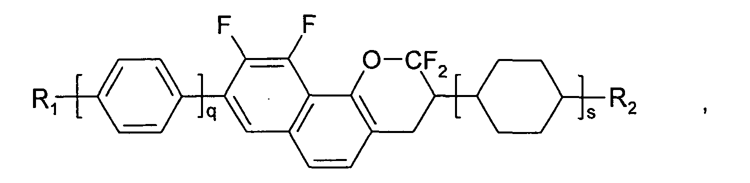 dans laquelle R₁, R₂, q et s présentent la signification indiquée.

6. Utilisation de composés de la formule I selon l'une des revendications précédentes en tant que composants de milieux cristallins liquides.

7. Milieu cristallin liquide comportant au moins deux composants cristallins liquides, **caractérisé en ce qu'**il comprend au moins un composé selon l'une des revendications 1 à 5.

8. Elément d'affichage électro-optique, **caractérisé en ce qu'**il contient un milieu cristallin liquide selon la revendication 7.
